# EUROPEAN PATENT APPLICATION

(11) **EP 4 000 524 A1**
(43) Date of publication of application: **25.05.2022**
(21) Application number: 20207265.8
(22) Date of filing: 12.11.2020
(51) Int. Cl.: A61B 6/00

(54) **PORTABLE X-RAY DETECTOR**

(71) Applicant: Koninklijke Philips N.V., 5656 AG Eindhoven (NL)
(72) Inventor: HERMANN, Eugen, 5656 AE Eindhoven (NL); KOEPNICK, Johannes, 5656 AE Eindhoven (NL)
(74) Representative: Philips Intellectual Property & Standards

(57) **Abstract**

The present invention relates to a portable X-ray detector (10), comprising:
- a housing (20);
- an X-ray detector array (30);
- a plurality of coloured LEDs (40) or other coloured light sources: and
- a processing unit (40);
wherein, the housing comprises a front side, and wherein the portable X-ray detector is configured such that X-rays to be detected impinge on the X-ray detector array at the front side of the housing;
wherein, the housing comprises a rear side opposite to the front side, and wherein the portable X-ray detector is configured such that the rear side is configured to be placed upon or placed against a support structure;
wherein, the housing comprises a plurality of edge sides connecting the front side to the rear side;
wherein, the plurality of coloured LEDs or other coloured light sources are integrated into at least one edge side, such that a centre axis of light emission for each LED or other coloured light source is directed backwards and outwards;
wherein, the processing unit is configured to determine a current state of the portable X-ray detector from a plurality of possible states of the portable X-ray detector;
wherein, the processing unit is configured to control the plurality of coloured LEDs or other coloured light sources to provide a light output indicative of the determined current state.

## Description

### FIELD OF THE INVENTION

The present invention relates to portable X-ray detector and to a portable X-ray detection system.

### BACKGROUND OF THE INVENTION

If a patient cannot be positioned on an X-ray table of a normal X-ray imaging system, a portable X-ray detector can be utilised. The portable X-ray detector can be used in a more convenient location, and the patient is positioned on the x-ray detector if the X-ray detector is positioned horizontally, or adjacent to the portable X-ray detector if the X-ray detector is positioned vertically. However, the majority if not all of the portable X-ray detector can be covered by the patient and/or the clothing, and an operator of the portable X-ray detector cannot then view any indicators of the status of the portable X-ray detector.

There is need to address this issue.

### SUMMARY OF THE INVENTION

It would be advantageous to have an improved portable X-ray detector with respect to how an operator can be provided with information relating to an internal state of the portable X-ray detector.

The object of the present invention is solved with the subject matter of the independent claims, wherein further embodiments are incorporated in the dependent claims. It should be noted that the following described aspects and examples of the invention apply also to the portable X-ray detector and to the portable X-ray detection system.

According to a first aspect, there is provided a portable X-ray detector, comprising:
- a housing;
- an X-ray detector array;
- a plurality of coloured LEDs or other coloured light sources: and
- a processing unit.

The housing comprises a front side, and the portable X-ray detector is configured such that X-rays to be detected impinge on the X-ray detector array at the front side of the housing. The housing comprises a rear side opposite to the front side, and the portable X-ray detector is configured such that the rear side is configured to be placed upon or placed against a support structure. The housing comprises a plurality of edge sides connecting the front side to the rear side. The plurality of coloured LEDs or other coloured light sources are integrated into at least one edge side, such that a centre axis of light emission for each LED or other coloured light source is directed backwards and outwards. The processing unit is configured to determine a current state of the portable X-ray detector from a plurality of possible states of the portable X-ray detector. The processing unit is configured to control the plurality of coloured LEDs or other coloured light sources to provide a light output indicative of the determined current state.

In other words, an operator of the portable X-ray detector facing the front side of the housing of the portable X-ray detector, is provided with information relating to the internal state of the X-ray detector even when the portable X-ray detector is in use and could be completely covered by someone positioned on the X-ray detector. This is because, coloured LEDs or other coloured light sources are located around an edge or indeed around all the edges of the housing of the X-ray detector, but projecting light backwards and to the sides. Thus, the support structure such as table, mattress, or indeed wall if the X-ray detector is positioned against a wall, is illuminated around the X-ray detector over a width and height greater than the physical size of the X-ray detector. Thus, even when the X-ray detector itself cannot be viewed, the operator can see this light projected around the X-ray detector that indicates the state of the X-ray detector.

Here, the other coloured light sources could be lasers, or any other light source such as a lamps configured to produce coloured light.

In an example, the centre axis of light emission for each LED or other coloured light source is at an angle of 5-80 degrees to a plane of the front side.

In an example, the centre axis of light emission for each LED or other coloured light source is at an angle of 10-60 degrees to a plane of the front side.

In an example, the centre axis of light emission for each LED or other coloured light source is at an angle of 30-60 degrees to a plane of the front side.

In an example, the centre axis of light emission for each LED or other coloured light source is at an angle of 30-45 degrees to a plane of the front side.

In an example, the plurality of possible states comprises two or more of: WI-FI status on; WI-FI status off; WI-FI status connected; WI-FI status connection issue; battery status fully or almost fully charged; battery status almost fully dis-charged; internal detector status ready; internal detector status image readout; internal detector status image transmission; internal detector status failure; internal detector status exposure; detector status standby.

In an example, the plurality of coloured LEDs or other coloured light sources are integrated into a plurality of edge sides.

In an example, each edge side comprises an equivalent set of coloured LEDs or other coloured light sources.

Thus, a light output indicating an internal state of the X-ray detector can be projected out and backwards from each side of the X-ray detector, such that the same information is provided in all directions, mitigating the circumstance when one or more edges are completely covered extending away from the detector, but at least one edge enables live projection to be viewed by the operator.

In an example, for each state of the plurality of states the processing is configured to provide a different light output.

In this manner, a very simple means is provided to provide information to the operator of the X-ray detector as to the internal state of the detector, depended upon a different light output that could for example be a colour, intensity, modulation, or combination of such.

In an example, the different light outputs comprise one or more of: colours, intensities of light, on-off modulation frequency, colour change, fading or non-fading.

In an example, the processing unit is configured to determine a second current state of the portable X-ray detector from the plurality of possible states of the portable X-ray detector, and wherein the processing unit is configured to control the plurality of coloured LEDs or other coloured light sources to provide a light output indicative of the determined second current state.

Thus for example the operator could be provided with a light output surrounding the X-ray detector that indicates that the battery level of the X-ray detector is low and therefore the operator will need to recharge the X-ray detector. However, at the same time the operator can be provided with light output surrounding the X-ray detector, that could be intermittently provided with the first light output, that indicates that the detector is being read out at that time. Thus, the operator could appreciate that once the examination is over the X-ray detector will need to be charged, and at the same time can take a close look at the data just being read out to ensure that the low charge level of the X-ray detector has not compromised image quality.

In an example, the processing unit is configured to control the plurality of coloured LEDs or other coloured light sources to alternate the provision of the light output indicative of the determined first current state and the determined second current state.

In a second aspect, there is provided a portable X-ray detection system, comprising:
- an X-ray source; and
- a portable X-ray detector according to the first aspect.

In an example, the system comprises a second portable X-ray detector according to the first aspect. The system is configured to control the processing units of the two portable X-ray detectors such that the plurality of coloured LEDs or other coloured light sources of each portable X-ray detector are configured to provide a light output indicate of the portable X-ray detector.

In this way, when more than one portable X-ray unit is being used in a system, the operator knows which colour relates to which portable X-ray detector. For example, the same state for both portable X-ray detectors can be represented by different light outputs.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a portable X-ray detector shown in plan view from the front and a cross-sectional side view;
Fig. 2 shows a schematic representation of an example of a portable X-ray detection system;
Fig. 3 shows examples of two portable X-ray detectors;
Fig. 4 shows an example of a portable X-ray detector with light being emitted backwards and outwards from the edges; and
Fig. 5 shows a cross sectional view of a part of a portable X-ray detector.

### DETAILED DESCRIPTION OF EMBODIMENTS

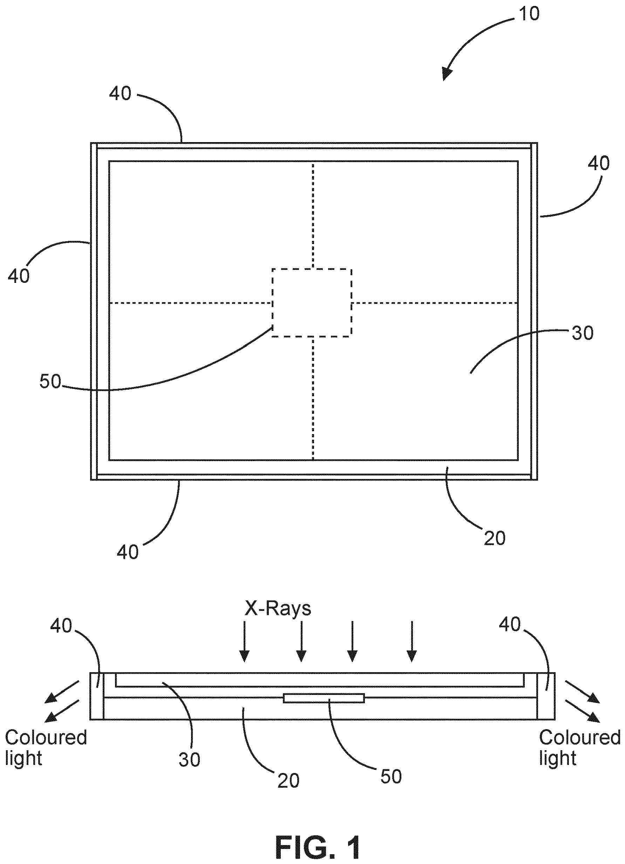

Fig. 1 shows an example of a portable X-ray detector 10. The portable X-ray detector comprises a housing 20, an X-ray detector array 30, a plurality of coloured LEDs or other coloured light sources 40, and a processing unit 40. The housing comprises a front side 22. The portable X-ray detector is configured such that X-rays to be detected impinge on the X-ray detector array at the front side of the housing. The housing also comprises a rear side 24 opposite to the front side. The portable X-ray detector is configured such that the rear side can be placed upon or placed against a support structure. The housing comprises a plurality of edge sides 60 connecting the front side to the rear side. The plurality of coloured LEDs or other coloured light sources are integrated into at least one edge side, such that a centre axis of light emission for each LED or other coloured light source is directed backwards and outwards. Here backwards and outwards is with respect to a viewer facing a centre of the front side of the housing. It is also to be noted that the front side of the housing could be in the form of an X-ray transparent material to protect the X-ray detector array, or could indeed have a region with no structural material (i.e., a hole) such that X-rays can directly interact with the X-ray detector array. The processing unit is configured to determine a current state of the portable X-ray detector from a plurality of possible states of the portable X-ray detector. The processing unit is configured also to control the plurality of coloured LEDs or other coloured light sources to provide a light output indicative of the determined current state.

According to an example, the centre axis of light emission for each LED or other coloured light source is at an angle of 5-80 degrees to a plane of the front side.

According to an example, the centre axis of light emission for each LED or other coloured light source is at an angle of 10-60 degrees to a plane of the front side.

According to an example, the centre axis of light emission for each LED or other coloured light source is at an angle of 30-60 degrees to a plane of the front side.

According to an example, the centre axis of light emission for each LED or other coloured light source is at an angle of 30-45 degrees to a plane of the front side.

According to an example, the plurality of possible states comprises two or more of: WI-FI status on; WI-FI status off; WI-FI status connected; WI-FI status connection issue; battery status fully or almost fully charged; battery status almost fully dis-charged; internal detector status ready; internal detector status image readout; internal detector status image transmission; internal detector status failure; internal detector status exposure; detector status standby.

According to an example, the plurality of coloured LEDs or other coloured light sources are integrated into a plurality of edge sides.

According to an example, each edge side comprises an equivalent set of coloured LEDs or other coloured light sources.

According to an example, for each state of the plurality of states the processing is configured to provide a different light output.

According to an example, the different light outputs comprise one or more of: colours, intensities of light, on-off modulation frequency, colour change, fading or non-fading.

According to an example, the processing unit is configured to determine a second current state of the portable X-ray detector from the plurality of possible states of the portable X-ray detector, and wherein the processing unit is configured to control the plurality of coloured LEDs or other coloured light sources to provide a light output indicative of the determined second current state.

According to an example, the processing unit is configured to control the plurality of coloured LEDs or other coloured light sources to alternate the provision of the light output indicative of the determined first current state and the determined second current state.

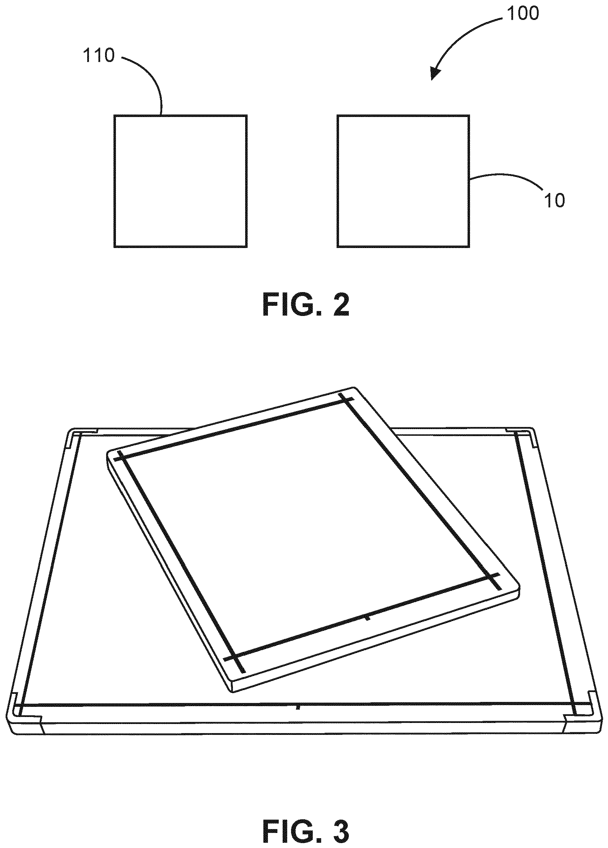

Fig. 2 shows an example of a portable X-ray detection system 100. The portable X-ray system comprises an X-ray source 110 and a portable X-ray detector 10 as described with respect to Fig. 1.

According to an example, the portable X-ray system comprises a second portable X-ray detector 10 as described with respect to Fig. 1. The system is configured to control the processing units of the two portable X-ray detectors such that the plurality of coloured LEDs or other coloured light sources of each portable X-ray detector are can provide a light output indicate of the portable X-ray detector.

The portable X-ray detector is now described in more detail, where reference is made to Figs. 3-5.

The inventors were seeking to solve a problem relating to utilisation of a portable X-ray detector, in that when in use an operator is frequently unable to see indicators that provide status information for the portable X-ray detector. Such indicators can be for example small LEDs that the operator can see directly when the portable X-ray detector is not in use. However, in use the patient and/or their clothing can obscure these indicators, and the operator is then unaware of the internal state of the portable X-ray detector, and/or the portable X-ray detector indicator or indicators are not visible due to a rotational positioning of the operator with respect to the portable X-ray detector. If for example, there is then a problem such as a Wi-Fi connection is lost, where the portable X-ray detector is placed under the patient and the indicator cannot be seen, then the operator receives no indication that there is a problem.

The inventors realised that a technology used, called Ambilight, for Philips TVs could be adapted and utilised in a completely new way with respect to portable X-ray detectors. The Ambilight lighting technology, utilises a series of LED lights set into the back of Philips TVs, which shine light onto the surrounding wall to extend picture beyond the TV bezels. This light can then for example follow the same hues as that shown on the TV.

The realisation that the inventors made was, that LEDs or other coloured light sources integrated into the edges of the portable X-ray detector could project light backwards and sideways (or outwards) away from the portable X-ray detector itself and not directly towards an operator who would be viewing towards the front of the portable X-ray detector. However, the light then projects upon background to the portable X-ray detector, such as a table or mattress if the portable X-ray detector is used horizontally or wall if the portable X-ray detector is used vertically. The light being projected onto the surroundings is then much more widely spaced than the portable X-ray detector itself, and even if the portable X-ray detector is totally covered an operator can still see the light projected onto this background.

Then, dependent upon the internal state of the portable X-ray detector, different light in terms of colour, intensity, or frequency modulation for example can then be utilised to relay to the operator the internal state of the portable X-ray detector.

Thus, the operator can be provided with information relating to for example that the detector is ready for acquisition, or the image is being read out or transmitted, or be provided with the Wi-Fi status or battery status of the detector. The operator can also be provided with light base feedback indicating that the detector has finished image read out, in order that the operator can prevent movements during the read-out phase that would otherwise lead to image artifacts by avoiding vibrations/movements during the read-out phase.

In other words, bias lighting is integrated into the back of the detector that illuminates the surrounding area with different light colours and/or intensities, and/or modulations that depend upon the internal state of the detector. The operator is then always provided with indications of the internal state of the detector even if detector itself is totally covered and cannot be seen by the operator.

Fig. 3 shows examples of portable X-ray detectors, with one placed upon the other.

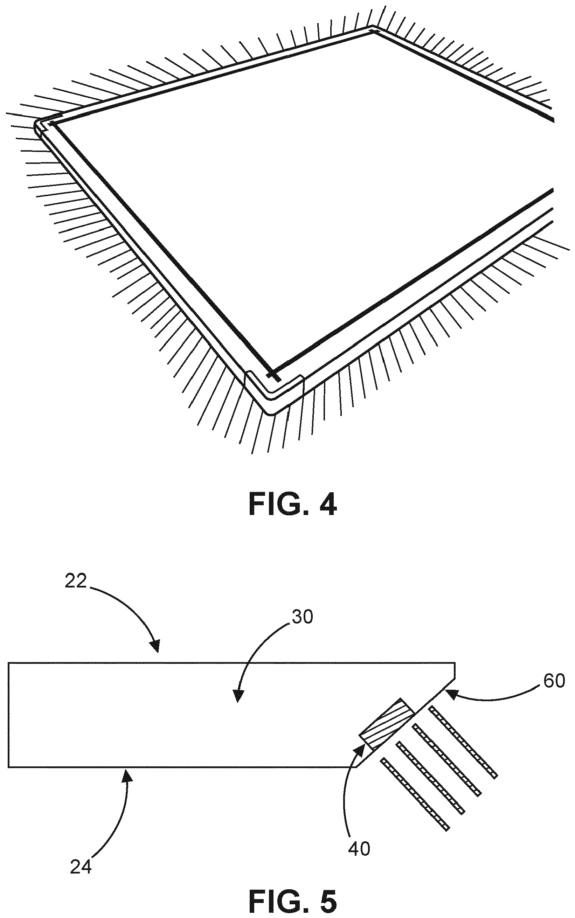

Fig. 4 shows a portable X-ray detector, where internal processor has determined that the portable X-ray detector is ready for acquisition. Accordingly, the internal processor is controlled series of LEDs or other coloured light sources arranged around the edges of the portable X-ray detector, which direct their light backwards and sideways, to provide a specific colour light projected onto the surroundings. This light being directed downwards and outwards is represented by the rays in the form of lines projecting downwards and outwards, which can for example be a coloured light - for example green.

Fig. 5 shows a cross-section through an end portion of a portable X-ray detector, showing how LEDs or other coloured light sources can be housed into an inclined edge of the housing of the portable X-ray detector in order that the light is projected backwards and sideways to illuminate the surroundings. This shows the housing 20, with a front side 22 and a rear side 24, and with an inclined edge side 60. One or more coloured LEDs or other coloured light sources 40 are integrated into the edge side to project light backwards and outwards in order to illuminate the surroundings upon with the rear side is laid upon or adjacent to.

Thus, the backward and outward facing LEDs or other coloured light sources are oriented to project an ambient light in different colours onto the surrounding area (table, patient mattress) beside the detector edges. The ambient light can be made to blink or change colours (fading or non-fading) depending on the state of the detector. Exemplar states and associated controlled LED or other coloured light source operation can be:
Off (no light)
Standby (no light - green faded)
Ready for exposure (e.g. green)
Internal failure or connection issue (e.g. red blinking)
Exposure (blue)
Readout (orange blinking)
Battery charge level low (yellow)

These are however only provided as examples, and many other possibilities of colours for different states and when intensity variations on modulations can be provided. Indeed, when the portable X-ray detector has two internal states at the same time, for example a charge level of the battery is low, and the detector is ready for exposure, then the lighting can automate between green and yellow to indicate that the device is ready for exposure but will need to be recharged before too long.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A portable X-ray detector (10), comprising:
- a housing (20);
- an X-ray detector array (30);
- a plurality of coloured LEDs or other coloured light sources (40): and
- a processing unit (40);
wherein, the housing comprises a front side (22), and wherein the portable X-ray detector is configured such that X-rays to be detected impinge on the X-ray detector array at the front side of the housing;
wherein, the housing comprises a rear side (24) opposite to the front side, and wherein the portable X-ray detector is configured such that the rear side is configured to be placed upon or placed against a support structure;
wherein, the housing comprises a plurality of edge sides (60) connecting the front side to the rear side;
wherein, the plurality of coloured LEDs or other coloured light sources are integrated into at least one edge side, such that a centre axis of light emission for each LED or other coloured light source is directed backwards and outwards;
wherein, the processing unit is configured to determine a current state of the portable X-ray detector from a plurality of possible states of the portable X-ray detector;
wherein, the processing unit is configured to control the plurality of coloured LEDs or other coloured light sources to provide a light output indicative of the determined current state.

2. Detector according to claim 1, wherein the centre axis of light emission for each LED or other coloured light source is at an angle of 5-80 degrees to a plane of the front side.

3. Detector according to claim 2, wherein the centre axis of light emission for each LED or other coloured light source is at an angle of 10-60 degrees to a plane of the front side.

4. Detector according to claim 2, wherein the centre axis of light emission for each LED or other coloured light source is at an angle of 30-60 degrees to a plane of the front side.

5. Detector according to claim 2, wherein the centre axis of light emission for each LED or other coloured light source is at an angle of 30-45 degrees to a plane of the front side.

6. Detector according to any of claims 1-5, wherein the plurality of possible states comprises two or more of: WI-FI status on; WI-FI status off; WI-FI status connected; WI-FI status connection issue; battery status fully or almost fully charged; battery status almost fully dis-charged; internal detector status ready; internal detector status image readout; internal detector status image transmission; internal detector status failure; internal detector status exposure; detector status standby.

7. Detector according to any of claims 1-6, wherein the plurality of coloured LEDs or other coloured light sources are integrated into a plurality of edge sides.

8. Detector according to claim 7, wherein each edge side comprises an equivalent set of coloured LEDs or other coloured light sources.

9. Detector according to any of claims 1-8, wherein for each state of the plurality of states the processing is configured to provide a different light output.

10. Detector according to claim 9, wherein the different light outputs comprise one or more of: colours, intensities of light, on-off modulation frequency, colour change, fading or non-fading.

11. Detector according to any of claims 1-10, wherein, the processing unit is configured to determine a second current state of the portable X-ray detector from the plurality of possible states of the portable X-ray detector, and wherein the processing unit is configured to control the plurality of coloured LEDs or other coloured light sources to provide a light output indicative of the determined second current state.

12. Detector according to claim 11, wherein the processing unit is configured to control the plurality of coloured LEDs or other coloured light sources to alternate the provision of the light output indicative of the determined first current state and the determined second current state.

13. A portable X-ray detection system (100), comprising:
- an X-ray source (110); and
- a portable X-ray detector (10) according to any of claims 1-12.

14. Portable X-ray detector system according to claim 13, wherein the system comprises a second portable X-ray detector (10) according to any of claims 1-12, wherein the system is configured to control the processing units of the two portable X-ray detectors such that the plurality of coloured LEDs or other coloured light sources of each portable X-ray detector are configured to provide a light output indicative of the portable X-ray detector.
